# EUROPEAN PATENT SPECIFICATION

(11) **EP 1 207 394 B1**
(45) Date of publication and mention of the grant of the patent: **30.12.2009**
(21) Application number: 01126829.9
(22) Date of filing: 10.11.2001
(51) Int. Cl.: G01N 33/569, G01N 33/573, G01N 33/543, G01N 33/58

(54) **Immunoassay for HIV protease inhibitors**
Immunoassay für HIV-Protease-Hemstoffe
Immunoessai pour les inhibiteurs de la protéase de VIH

(30) Priority: 14.11.2000 US 712525
(43) Date of publication of application: 22.05.2002
(73) Proprietor: Roche Diagnostics GmbH, 68305 Mannheim (DE); F. Hoffmann-La Roche AG, 4002 Basel (CH)
(72) Inventor: Salamone, Salvatore J., Stockton, NJ 08559 (US); Sigler, Gerald, Carmel, IN 46033 (US); Arabshahi, Lili, Carmel, IN 46032 (US)

(56) References cited:
- US-A- 4 134 792
- US-A- 6 107 052
- YU, SUNG-LIANG ET AL: "Assay of HIV-1 protease activity by use of crude preparations of enzyme and biotinylated substrate" JOURNAL OF VIROLOGICAL METHODS, vol. 53, 1995, pages 63-73, XP001069545
- MANSFELD, H-W ET AL: "Detection of inhibition of HIV-1 protease activity by an enzyme-linked immunosorbent assay (ELISA)" JOURNAL OF IMMUNOLOGICAL METHODS, vol. 161, 1993, pages 151-155, XP001069544
- SARUBBI E ET AL.: "A high throughput assay for inhibitors of HIV-1 protease." FEBS LETT., vol. 279, 1991, pages 265-269, XP002197217
- WILTSHIRE, HR ET AL.: "Chromatographic and immunochemical approaches to the analysis of the HIV protease inhibitor saguinavir in plasma" ANALYTICAL BIOCHEMISTRY, vol. 281, 2000, pages 105-114, XP002196958
- THERAPEUTIC DRUG MONITORING, vol. 22, 2000, pages 465-473, XP002196959

## Description

### BACKGROUND OF THE INVENTION

This invention relates generally to the field of measuring an analyte in a liquid medium. More specifically, it relates to immunoassay methods for the measurement of therapeutic drugs in biological samples. In particular, the invention relates to non-isotopic immunoassay methods for the detection of protease inhibitors, especially HIV protease inhibitors, in biological samples.

HIV protease inhibitors are an important new class of drugs which have made a significant impact on the health care of AIDS patients since the first one, saquinavir, was introduced to the marketplace in 1995. Examples of other protease inhibitors include amprenavir, indinavir, nelfinavir and ritonavir. They are especially effective in combination with other anti-HIV drugs such as reverse transcriptase inhibitors or with other HIV protease inhibitors. In spite of remarkable success with these new therapeutic regimens, there are strong indications that results would be much improved if therapeutic drug testing methods were available for monitoring protease inhibitors. Not all patients respond optimally to the protease inhibitor combination therapies. And even those who do respond initially can develop drug resistance due to the notoriously high rate of mutation of the HIV virus. However, it has been shown that there is a clear relationship between plasma levels of the protease inhibitors and therapeutic efficacy based upon decreased viral load and increased CD4 cell count. One problem lies in the fact that the drugs are metabolized extensively and are subject to complex drug-drug interactions. This results in extremely complex pharmacokinetics and a strong element of unpredictability between dosage and resultant drug levels at any particular time for any particular patient. With therapeutic drug monitoring, drug dosages could be individualized to the patient, and the chances of keeping the virus in check would be much higher. But routine therapeutic drug monitoring of protease inhibitors would require the availability of simple automated tests adaptable to high throughput clinical analyzers. Currently most reports on therapeutic drug monitoring of protease inhibitors have used HPLC methods which are slow, labor-intensive, and expensive. Recently there was a report of a radioimmunoassay (RIA) method for saquinavir. However, such a method would not be adaptable to high throughput therapeutic drug monitoring and, like all RIA methods, suffers from the disadvantages of regulatory, safety and waste disposal issues relating to the radioactive isotope label used in the assay. The most desirable assay formats for therapeutic drug monitoring, therefore, are non-isotopic immunoassays, and such methods have heretofore been unknown for monitoring HIV protease inhibitors.

HPLC has been the method of choice for monitoring HIV protease inhibitors. Two recent reports in the literature describe HPLC assays for the simultaneous determination of several protease inhibitors in human plasma, Poirier et al., Therapeutic Drug Monitoring 22, 465-473, 2000 and Remmel et al., Clinical Chemistry 46, 73-81, 2000. There is only one known report of an immunoassay of any sort for HIV protease inhibitors. Described earlier this year was an RIA for saquinavir, its use with patient samples, and a comparison with HPLC methods, Wiltshire et al., Analytical Biochemistry 281, 105-114, 2000. There was no teaching or suggestion of non-isotopic alternatives, however.

Yu et al., J. of Virological Methods 53, 63-73, 1995, describe an enzyme immunoassay for monitoring protease reactions of human immunodeficiency virus using a gag fusion protein as a substrate for the HIV protease. The analyte to be determined thus is HIV protease, not a single specific HIV protease inhibitor.

Mansfeld et al., J. of Immunological Methods 161, 151-155, 1993, disclose an ELISA for the detection of inhibition of HIV-1 protease activity. Similarly to Yu et al., in this screening assay a fusion protein containing HIV proteins gp41 and p24 is coated on a solid phase serving as a substrate for HIV protease. HIV protease is added and cleaves the fusion protein. As a result, an antibody which is directed against the p24 cannot bind anymore to the proteolytically deleted fusion protein. In the presence of an HIV protease inhibitor the proteolytic cleavage by HIV protease is inhibited and thus the antibody specific to p24 can bind to the intact gp41-p24 fusion protein. The binding of the anti-p24 antibody can be detected by a further labeled antibody that binds specifically to the anti-p24 antibody. Mansfeld et al. use an antibody as a receptor which is specific for an HIV protease substrate (gp41-p24), not for an inhibitor. Addition of any HIV protease inhibitor will prevent cleavage of the substrate and therefore maintain the antibody recognition. Thus the HIV protease inhibitor is measured indirectly and non-specifically.

A similar screening assay has been described by Sarubbi et al., FEBS Letters 279/2, 265-269, 1991.

Chemical and biological assays generally involve contacting the analyte of interest with a pre-determined, non-limiting amount of one or more assay reagents, measuring one or more properties of a resulting product (the detection product), and correlating the measured value with the amount of analyte present in the original sample, typically by using a relationship determined from standard or calibration samples containing known amounts of analyte of interest in the range expected for the sample to be tested. Typically, the detection product incorporates one or more detectable labels which are provided by one or more assay reagents. Examples of commonly used labels include radioactive isotope labels such as ¹²⁵I and ³²P, enzymes such as peroxidase and beta-galactosidase and enzyme substrate labels, fluorescent labels such as fluoresceins and rhodamines, electron-spin resonance labels such as nitroxide free radicals, immunoreactive labels such as antibodies and antigens, labels which are one member of a binding pair such as biotin-avidin and biotin-streptavidin, and electrochemiluminescent labels such as those containing a ruthenium bipyridyl moiety. Sandwich assays typically involve forming a complex in which the analyte of interest is sandwiched between one assay reagent which is ultimately used for separation, e.g., antibody, antigen, or one member of a binding pair, and a second assay reagent which provides a detectable label. Competition assays typically involve a system in which both the analyte of interest and an analog of the analyte compete for a binding site on another reagent, e.g., an antibody, wherein one of the analyte, analog or binding reagent possesses a detectable label.

### SUMMARY OF THE INVENTION

The present invention comprises a method of immunoassay for an HIV protease inhibitor which comprises the steps of incubating a sample suspected of containing the protease inhibitor with a receptor specific for the inhibitor and a non-isotopic conjugate comprised of a ligand or analog of the inhibitor and a non-isotopic label, measuring the amount of receptor that binds to the conjugate, and correlating the amount of bound partner to the amount of protease inhibitor in the sample as defined in the claims. The incubation of sample with receptor and conjugate can be done sequentially or simultaneously. The sample is preferably a bodily fluid such as whole blood, serum, plasma, urine, saliva, cerebrospinal fluid, or tears. The receptor or binding partner may be an antibody selective for a particular protease inhibitor over other protease inhibitors, protease inhibitor metabolites, or co-administered non-protease inhibitor drugs. Alternatively, in another aspect of the invention, the receptor is an antibody reactive with a class of structurally related protease inhibitors and/or protease inhibitor metabolites. The non-isotopic conjugate is a covalent or non-covalent complex of a non-isotopic label with a ligand selected from the group consisting of protease inhibitors, protease inhibitor derivatives and protease inhibitor analogs. Examples of non-isotopic labels include enzymes, fluorogenic compounds, chemiluminescent materials, electrochemical mediators, particles, reporter groups such as biotin, enzyme inhibitors such as mycophenolic acid, and macromolecular carriers such as proteins, glycoproteins, complex polysaccharides and nucleic acids. The immunoassay may be performed in a heterogeneous format utilizing a solid phase or in a homogeneous format using a solution or suspension, both of which assay formats are well known in the art. One preferred heterogeneous format is a microtiter plate ELISA (enzyme-linked immunosorbent assay). Preferred homogeneous formats include microparticle agglutination and uncompetitive inhibition immunoassays, e.g., the mycophenolic acid/inosine monophosphate dehydrogenase method described in Dorn et al., US Serial No. 09/603,646 filed June 26, 2000.

### BRIEF DESCRIPTION OF THE DRAWINGS

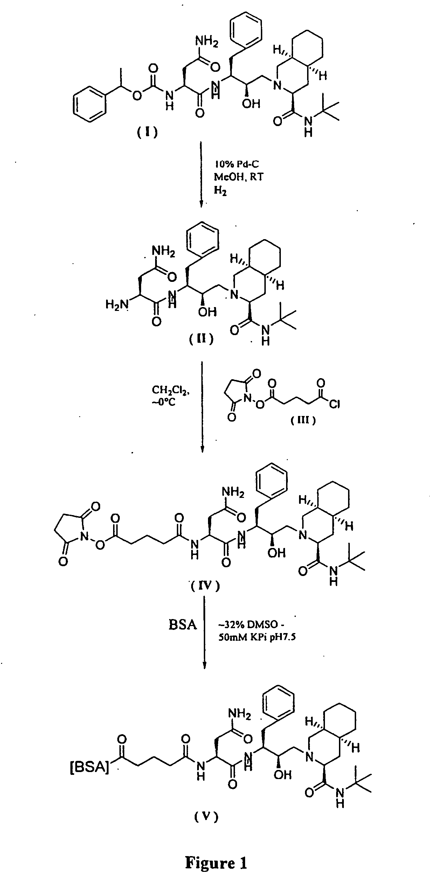

Figure 1 is a schematic representation of the synthesis of [cis-1-oxo-4-{1(S)-[1(S)-benzyl-3-[3 (S)-tert-butylcarbamoyl-decahydro-(4aS,8aS)-isoquinolin-2-yl]-2(R)-hydroxy-propylcarbamoyl]-2-carbamoyl-ethylcarbamoyl}-butyl]-BSA as described in Examples 1-3.
Figure 2 is a graph prepared by plotting the results obtained in Example 6 in which samples containing various concentrations of saquinavir were assayed according to the present invention. Concentration of saquinavir is plotted on the X-axis and absorbance at 450 nm is plotted on the Y-axis.

### DETAILED DESCRIPTION OF THE INVENTION

Non-isotopic immunoassays for HIV protease inhibitors may be constructed in heterogeneous or homogeneous formats. Heterogeneous immunoassays are distinguished by incorporating a solid phase separation of bound analyte from free analyte or bound label from free label. Solid phases can take a variety of forms well known in the art, including but not limited to tubes, plates, beads and strips. One particularly preferred form is the microtiter plate. The solid phase material may be comprised of a variety of glasses, polymers, plastics, papers, or membranes. Particularly preferred are plastics such as polystyrene. Heterogeneous immunoassays may be competitive or non-competitive, i.e., sandwich, formats.

For low molecular weight analytes such as HIV protease inhibitors, selected from the group consisting of saquinavir, amprenavir, indinavir, nelfinavir and ritonavir, competitive formats are preferred. Competitive heterogeneous immunoassays for HIV protease inhibitors may be formatted in various ways. For example, in one format, an antibody to a protease inhibitor is immobilized on a solid phase followed by incubation with sample and conjugate, which compete for a limited number of receptor binding sites. The unbound portion of the analyte and conjugate is then removed, and the amount of bound conjugate is measured. The amount of bound conjugate is inversely proportional to the amount of HIV protease inhibitor in the sample. A dose-response calibration curve is constructed using known amounts of HIV protease inhibitor using methods that are well-known in the art.

A second preferred format for the present invention involves first preparing a conjugate of an HIV protease inhibitor derivative with a macromolecular carrier substance such as a protein. The preparation of such a conjugate is described herein in Examples 1-3 for a saquinavir derivative conjugate with the carrier protein bovine serum albumin (BSA). Conjugates of this type may be immobilized on a solid phase of choice using covalent or passive immobilization. In Example 4, passive immobilization on a microtiter plate is illustrated. Following preparation of the conjugate-coated plate, receptor is added at a pre-determined optimal dilution as well as sample containing HIV protease inhibitor. A competition results between the solid phase bound conjugate and the HIV protease inhibitor in solution for a limited number of receptor binding sites. After incubation, the solid phase is washed to remove unbound receptor. Finally a label is added which is used to detect the presence of bound antibody. In the case of an ELISA assay such as desecribed in Example 6, the label includes a secondary antibody or receptor directed against the species of the bound receptor, e.g., rabbit anti-sheep antibody, which is conjugated to an enzyme label, e.g., horseradish peroxidase (HRP). Other enzyme labels and secondary binding substances will be readily apparent to those skilled in the art of microtiter plate ELISAs. Similarly to the first described assay format, the amount of bound enzyme conjugate is inversely proportional to the amount of HIV protease inhibitor in the sample. A dose-response calibration curve is constructed with known amounts of HIV protease inhibitor, and the amount of HIV protease inhibitor in the unknown sample is then correlated to the calibration curve using standard methods. The amount of bound conjugate is inversely proportional to the amount of HIV protease inhibitor in the sample.
A preferred embodiment of the present invention is a non-isotopic immunoassay for determining the presence or amount of an HIV protease inhibitor in a sample comprising the steps of combining a sample suspected of containing said protease inhibitor with a conjugate comprising a ligand of said protease inhibitor and mycophenolic acid, a receptor specific for said protease inhibitor, inosine-5'-monophosphate (IMP), nicotinamide adenine dinucleotide (NAD) and inosine-5'-monophosphate dehydrogenase (IMPDH), monitoring the production of reduced nicotinamide adenine dinucleotide (NADH), and correlating the production of NADH with the presence or amount of said protease inhibitor in said sample.

A preferred homogeneous microparticle immunoassay method and test kit of the present invention comprises a two-reagent system comprising ready-to-use liquid reagents for the detection of HIV protease inhibitors in serum, plasma, whole blood, urine and saliva. Kinetic interaction of microparticles in a solution is conveniently measured using automated analyzers. In this particular assay format, antibody against a specific protease inhibitor is loaded on the microparticle using covalent or passive immobilization, and the protease inhibitor derivative is linked to a macromolecule of choice such as aminodextran, which is then preferred to as a drug conjugate. A competitive reaction takes place between the drug conjugate and any drug in the serum sample for binding to a limited amount of specific antibody binding sites on the microparticles. The kinetic interaction of microparticles in solution is induced by binding of drug conjugate to the antibody on the microparticle and is inhibited by the presence of drug in the sample. The interaction of the microparticles is measured by the absorbance of the solution, which in turn is related to the turbidity of the solution. Cross-linking of particle and drug conjugate leads to higher turbidity (higher absorbance). Free drug binding to antibody on particles results in lower turbidity (lower absorbance).

A second format for a homogeneous microparticle immunoassay method and test kit comprises ready-to-use liquid reagents for the detection of HIV protease inhibitors in serum, plasma, whole blood, urine and saliva. Kinetic interaction of microparticles in a solution is conveniently measured using automated analyzers. In this assay format, a drug derivative linked to a macromolecule of choice such as bovine serum albumin is loaded on the microparticles using covalent or passive immobilization. Antibody against the specific protease inhibitor is formulated in a buffer system. A competitive reaction takes place between the drug conjugate on the microparticles and any drug present in serum sample for binding to a limited amount of specific antibody in the reaction solution. The kinetic interaction of microparticles in solution is induced by binding of drug-conjugate to the antibody and is inhibited by the presence of drug in the sample. The interaction of the microparticles is measured by the absorbance of the solution, which in turn is related to the turbidity of the solution. Cross-linking of particles and drug conjugate leads to higher turbidity (higher absorbance). Free drug binding to antibody on particles results in lower turbidity (lower absorbance).

In another immunoassay format of the present invention, a fluorescent polarization immunoassay method and test kit comprises ready-to-use liquid reagents for the detection of HIV protease inhibitors in serum, plasma, whole blood, urine and saliva, using the principle of fluorescence polarization. In this assay format, drug derivative is tagged or labeled with a fluorophore, and the antibody against the specific protease inhibitor is formulated in a buffer system. A competitive reaction takes place between the drug with the fluorescence tracer and any drug in serum sample for binding to a limited amount of specific antibody in the reaction solution.

When a fluorescent molecule, or fluorophore, is irradiated with light of the proper wavelength (excitation wavelength) some of the light is emitted, although at a longer wavelength (emission wavelength). Whether or not the emitted light is polarized depends on the freedom of the fluorophore to rotate in solution. A small molecule, such as fluorescein, can rotate rapidly before light emission occurs, resulting in depolarization of the emitted light. In contrast, a fluorescent macromolecule, such as a fluorescein-labeled protein, will rotate much more slowly. Thus, in the time frame between excitation and emission, the macromolecule will have rotated only very slightly, and the emitted light will be polarized. Fluorescence polarization is a reproducible function of the drug concentration and is suitable for the quantitative determination of drug concentrations in samples.

Another immunoassay format contemplated by the present invention is a homogeneous electrochemical immunoassay based on the use of electroactive labels that are inhibited when bound to an antibody or other binding receptor. The preferred electroactive labels are reversible redox labels such as bipyridyl osmium complexes. Signal amplification can be achieved by redox cycling of these mediators bioelectrocatalytically by using a redox enzyme or through the use of an interdigitated array (IDA) electrode. The format used for the homogeneous assay is a sequential binding inhibition. The sample being assayed is mixed with the antibody or other binding receptor. If antigen is present, binding occurs. Any remaining unbound antibody/binding receptors are then mixed with the antigen labeled electroactive label. The unbound antigen labeled electroactive compounds are then measured at the electrode surface.

When no analyte is present in the sample, a greater amount of antibody or binding receptor will bind to the antigen-labeled electroactive compound. This results in maximum inhibition of the electroactive compound. High analyte concentrations in the sample result in little or no inhibition of the electroactive compound. Therefore, there is a positive correlation between electrochemical response and analyte concentration.

In yet another immunoassay format of the present invention, the analyte present in the sample competes with analyte-enzyme conjugate for binding sites on antibodies which are immobilized on capillary surfaces. The unbound analyte-enzyme conjugate flows to a detection zone where the enzyme turns the substrate into electroactive product. The product is then detected electrochemically at the electrode. When analyte concentration in the sample is high, there is more analyte-enzyme conjugate left unbound to flow to the detection zone. This results in a higher concentration of electroactive product produced by enzyme conjugate and a higher current detected at the electrode. Therefore, there is a positive correlation between current detected at the electrode and analyte concentration.

Another aspect of the present invention relates to kits useful for conveniently performing the assay methods of the invention for the determination of an HIV protease inhibitor. To enhance the versatility of the subject invention, reagents useful in the methods of the invention can be provided in packaged combination, in the same or separate containers, in liquid or lyophilized form so that the ratio of the reagents provides for substantial optimization of the method and assay. The reagents may each be in separate containers, or various reagents can be combined in one or more containers depending on cross-reactivity and stability of the reagents.
The test kit of the present invention comprises a receptor selected from the group consisting of antibodies and antibody fragments. This receptor is bound either directly or indirectly to a solid phase. Furthermore, the test kit of the present invention is useful for the determination of protease inhibitor selected from the group of saquinavir, amprenavir, indinavir, nelfinavir and ritonavir. In addition, the signal generating moiety of this test kit is selected from the group consisting of enzymes, fluorogenic compounds, chemiluminescent materials, electrochemical mediators, particles, reporter groups, enzyme inhibitors, and polypeptide carriers.

The reagent kit of the present invention comprises a receptor specific for an HIV protease inhibitor and a conjugate comprising a ligand of the inhibitor and a non-isotopic signal generating moiety. The reagents may remain in liquid form or may be lyophilized. The kit can further comprise calibration and control materials useful in performing the assay. The receptor or the conjugate may be immobilized on a solid support.

Any sample that is reasonably suspected of containing the analyte, i.e., an HIV protease inhibitor or metabolite, can be analyzed by the method of the present invention. The sample is typically an aqueous solution such as a body fluid from a host, for example, urine, whole blood, plasma, serum, saliva, semen, stool, sputum, cerebral spinal fluid, tears, mucus or the like, but preferably the sample is plasma or serum. The sample can be pretreated if desired and can be prepared in any convenient medium that does not interfere with the assay. An aqueous medium is preferred.
Antibody, or preferably, receptor, means a specific binding partner of the analyte and is any substance, or group of substances, which has a specific binding affinity for the ligand to the exclusion of other substances. Furthermore, said receptor is selected from the group consisting of antibodies and antibody fragments. In addition, said receptor is bound either directly or indirectly to a solid phase.

Ligand means any substance, or group of substances, which behaves essentially the same as the analyte with respect to binding affinity of the antibody for the analyte and is meant to include any HIV protease inhibitor or derivative and isomers thereof.

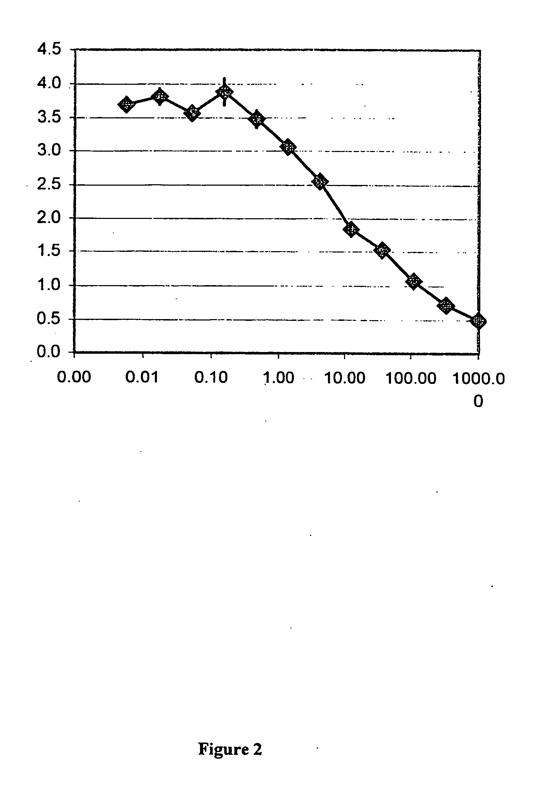

Calibration material means any standard or reference material containing a known amount of the analyte to be measured. The sample suspected of containing the analyte and the calibration material are assayed under similar conditions. Analyte concentration is then calculated by comparing the results obtained for the unknown specimen with results obtained for the standard. This is commonly done by constructing a calibration or dose response curve such as in Figure 2.

Various ancillary materials will frequently be employed in an assay in accordance with the present invention. For example, buffers will normally be present in the assay medium, as well as stabilizers for the assay medium and the assay components. Frequently, in addition to these additives, additional proteins may be included, such as albumin, or surfactants, particularly non-ionic surfactants, or the like.

It is to be understood that any reference throughout the specification and claims to an HIV protease inhibitor is meant to include the inhibitor as well as its biologically active and therapeutically active metabolites and derivatives which behave in a biological sense as the inhibitor.

The term derivative refers to a chemical compound or molecule made from a parent compound or molecule by one or more chemical reactions.

As used herein, a detector molecule, label or tracer is a non-radioactive identifying tag which, when attached to a carrier substance or molecule, can be used to detect an analyte. A label may be attached to its carrier substance directly or indirectly by means of a linking or bridging moiety. Examples of labels include enzymes such as β-galactosidase and peroxidase, fluorescent compounds such as rhodamine and fluorescein isothiocyanate (FITC), and luminescent compounds such as dioxetanes and luciferin.

### Example 1. Preparation of 2-[3(S)-[(L-asparaginyl)amino]-2(R)-hydroxy-4-phenylbutyl]-N-tert-butyl-decahydro-(4aS,8aS)-isoquinoline-3(S)-carboxamide (II)

To a solution of 548 mg of cis-2-[3(S)-[[N-(benzyloxycarbonyl)-L-asparaginyl]amino]-2(R)-hydroxy-4-phenylbutyl]-N-tert-butyl-decahydro-(4aS,8aS)-isoquinoline-3(S)-carboxamide (**I**, US Patent No. 5,196,438) in 50 ml of methanol in a flask was added 58 mg of 10% palladium on carbon. The flask and contents were placed under a hydrogen atmosphere (purge-evacuate cycle 5 times) and the contents then left under 1-2 atmospheres pressure of hydrogen overnight at room temperature with stirring. Thin layer chromatography analysis (silica gel plates, eluting with 10% methanol-chloroform) followed by staining of the plate in an iodine chamber indicated disappearance of starting material with a new, more polar spot appearing. The reaction was filtered through a pad of celite, washing with methanol, and the collected filtrates evaporated under reduced pressure. The residue was redissolved in a little methanol, filtered (0.2 µ, Gelman Acrodisc) and evaporated to dryness. The residue was redissolved in distilled methylene chloride and re-evaporated (repeated 5 times) and the residue dried under high vacuum for 2 days to give 451 mg of the product (**II**) as a white/off-white solid. ¹H-NMR: compatible. FAB (+) MS: 516 (M+H).

### Example 2. Preparation of cis-4-{1(S)-[1(S)-benzyl-3-[3(S)-tert-butylcarbamoyl-decahydro-(4aS,8aS)-isoquinolin-2-yl]-2(R)-hydroxy-propylcarbamoyl]-2-carbamoyl-ethylcarbamoyl}-butyric acid 2,5-dioxo-pyrrolidin-1-yl ester (IV)

To a stirred solution of 50 mg of (**II**) in 10 ml of dry methylene chloride (distilled from calcium hydride under argon) under argon and cooled in an ice water bath was added 24 mg, 1 molar equivalent, of 5-[(2,5-dioxo-1-pyrrolidinyl)oxy]-5-oxo-pentanoyl chloride (**III**, prepared similarly to European patent application EP 503454 and US Patent No. 5,248,611) as a solid in one lot. The reaction was stirred for 2 hours while maintaining cooling. Thin-layer chromatography (silica gel plates, eluting with 10% methanol-methylene chloride) indicated the reaction was complete. The reaction was diluted with methylene chloride, poured into 0.1 N aqueous hydrochloric acid, the pH adjusted to about 5 with 10% sodium carbonate, the mixture shaken well and the phases separated. The aqueous layer was further basified with 10% sodium carbonate to pH about 7 and re-extracted with methylene chloride. The combined organic extracts were washed with water (1 time), saturated aqueous sodium bicarbonate (3 times), saturated aqueous sodium chloride (1 time), dried (sodium sulfate), filtered and evaporated. The gel-like residue was dried under high vacuum at room temperature for several hours to give 57 mg of the product (**IV**) as a white solid and as a partial solvate with methylene chloride. ¹H-NMR: compatible. FAB (+) MS: 727 (M+H). HR (+) FAB MS: calculated (M+H) 727.4031, observed 727.4013.

### Example 3. Preparation of [cis-1-oxo-4-{1(S)-[1(S)-benzyl-3-[3(S)-tert-butylcarbamoyl-decahydro-(4aS,8aS)-isoquinolin-2-yl]-2(R)-hydroxy-propylcarbamoyl]-2-carbamoyl-ethylcarbamoyl}-butyl]-BSA (V)

To a stirring solution of 380 mg of bovine serum albumin (Miles-Pentex, Fraction V) in 7.6 ml of 50 mM potassium phosphate (KPi) pH 7.5 cooled in an ice water bath was added 1.9 ml of dimethylsulfoxide (DMSO) dropwise over 5-10 minutes. 1 ml of the resulting clear solution was withdrawn and kept as the BSA control. To the remaining solution of about 340 mg of BSA in 20% DMSO-50 mM KPi, pH7.5 was added 7.8 mg, about 2.1 molecular equivalents, of (**IV**) dissolved in a total of about 1.5 ml of DMSO, resulting in a reaction of (**IV**) with BSA in about 32% DMSO-50 mM KPi, pH7.5. The reaction was stirred overnight, allowing the temperature to attain room temperature. The resulting solution was transferred to a 3-15 ml capacity dialysis cassette (Pierce Chemical Co., Slide-A-Lyzer®, 10K molecular weight cutoff) and dialyzed sequentially at room temperature against 1 L each of 30%, then 20%, then 10% DMSO-50 mM KPi, pH7.5 for about 2 hours each, then against 50 mM KPi, pH7.5 at room temperature overnight followed by 50 mM KPi, pH7.5 (5 changes) over 3 days. The retentate was withdrawn from the cassette to give 19 ml of the conjugate (**V**) as a clear, essentially colorless, solution. The protein concentration was determined (Coomassie Blue, modified Bradford method) to be 18.6 mg/ml, using the BSA control as the standard.

### Example 4. Concentration ranges of antiserum and saquinavir-BSA conjugate

Corning micro-ELISA plates (96 wells) were used throughout. The saquinavir-BSA conjugate was diluted to 2 µg/ml in 0.1 M sodium carbonate buffer, pH 9.5. One hundred microliters of the buffer was placed in the wells of each row of the plate except the first row. The first row was filled with 200 µl of the 2 µg/ml saquinavir-BSA solution, and a twelve-tip micropipettor was used to transfer 100 µl from the first row to the second row of all columns at the same time. Mixing of contents of the second row was accomplished by re-pipetting 3 times. One hundred microliters from the second row was then transferred to the third row and mixed. This was repeated to the last row of the plate. After mixing, 100 µl was removed from the last row and discarded. The plate was placed in a humidified Zip-Loc bag and incubated for 1 hour at 37 °C.

After incubation, the plate was removed from the incubator and bag and emptied into a waste container. PostCoat solution was added to each well in the amount of 200 µl. This solution consisted of 1% gelatin hydrolysate, 2% sucrose, 0.15 M Tris, pH 7.4, 0.17% Tween 20, and 0.02% Thimerosal preservative. All reagents were from Sigma Chemicals. The plate was returned to the humidified bag and placed in the incubator for 1 hour.

During the incubation, saquinavir antiserum dilutions (from H.R. Wiltshire, see Anal. Biochem. 281, 105-114, 2000) were prepared as follows. A vial of serum was thawed in warm water, and 2 µl of 10% Thimerosal was added as a preservative. This was gently mixed so as not to form a foam. A Falcon flexible microtiter plate was used to prepare the serum dilutions by adding 110 µl of PBS-Tween (phosphate buffered saline containing 0.2% Tween 20) into each well except the first column of wells. To the first column of wells were added 110 µl of a 1:100 dilution of serum in PBS-Tween. To the second column, 110 µl of 1:100 dilution was also added using an eight place micropipettor. This was mixed by re-pipetting three times, then transferring 110 µl from the second column to the third and repeating the mixing. This was repeated for each column across the plate.

After the incubation of the coated plate was complete, it was emptied by washing with PBS-Tween and a final aspiration. Using an 8-place micropipettor, 100 µl was transferred from column 12 of the dilution plate to column 12 of the coated plate. Then 100 µl was transferred from column 11, etc. After transfer of diluted antibody was completed, the coated plate was re-bagged and incubated for 1 hour at 37 °C.

Five minutes before the incubation was complete, Zymed rabbit anti-sheep IgG-HRP conjugate was diluted 1:2000 in PBS-Tween and vortexed gently to completely mix. Upon completion of the hour incubation, the plate was washed 4 times with 300 µl of PBS-Tween using a BioTek Instruments, Inc. EL404 plate washer. One hundred microliters of the diluted rabbit anti-sheep IgG-HRP conjugate was then pipetted into each well, the plate re-bagged and incubated for 1 hour.

At the completion of the hour, the plate was removed from the incubator and bag and washed six times on the plate washer. One hundred microliters of K-BLUE enzyme substrate (Neogen, Inc.) was then added to each well and color allowed to develop in the dark for 5 minutes. Color development was halted by the addition of 100 µl of 1 N hydrochloric acid to each well. The optical density of each well was measured at two wavelengths, 405 nm and 450 nm using a Molecular Devices, Inc. ThermoMax plate reader. The readings at 450 nm showed that the measurement capability of the reader was exceeded at the higher concentrations of conjugate and antiserum. Therefore, the OD₄₀₅ measurements were used to calculate the extrapolated OD₄₅₀ by the well-known method of using a linear least squares regression of readings at both wavelengths.

The resulting data was analyzed by plotting the optical density for each concentration of saquinavir-BSA on the Y and X axes, respectively, for each dilution of the antiserum. This produced a set of 12 curves. Next, the optical density versus the dilution of serum was plotted for each concentration of saquinavir-BSA, producing a graph with 8 curves. The latter graph revealed that there was a rather pronounced pro-zone effect for concentrations of saquinavir-BSA of 0.125 µg/ml and below when combined with dilutions of the anti-saquinavir serum of 1:1000 or less. From these two graphs, it was decided that the combination of 62.5 ng/ ml conjugate and 1:12,800 dilution of antiserum would be used.

### Example 5. Assay specificity determination

An ELISA plate was coated with the above concentrations of saquinavir-BSA using the same conditions as set forth above. While the plates were being PostCoated, serial three-fold dilutions of saquinavir, ritonavir, indinavir, and nelfinavir were prepared in a 1 ml capacity 96-well plate. All drugs were prepared at 1 mg/ml in absolute methanol and stored at 4 °C until used. Briefly, 1 µl of each drug was transferred to wells of the first row of the plate, which contained 500 µl of PBS-Tween in each well of the first row and 200 µl of the same buffer in all other wells of those columns. After mixing, 100 µl from the first row of each of the four columns was transferred to the second row and mixed. This was repeated across each row until the seventh row was completed. The eighth row contained only buffer; this was the zero concentration of each drug.

Upon completion of the PostCoat incubation, the coated plate was washed and 50 µl of solution was transferred from row H of the dilution plate to row H of the coated plate; this was repeated from row G to row A using a micropipettor equipped with 4 tips. Upon completion of this, 50 µl of saquinavir antiserum, diluted 1:12,800 in PBS-Tween, was added to each well of the coated plate. The plate was incubated as above. After 1 hour, the plate was washed 4 times, and 100 µl of 1:2,000 Zymed rabbit anti-sheep IgG-HRP diluted in PBS-Tween was added and the plate incubated as above. The plate was processed further as described above. Readings of the optical density at 450 nm showed a dose-response curve for saquinavir and a lesser response for nelfinavir; no dose-response curves were observed for the other drugs. From these data it was calculated that the antiserum cross-reacted with nelfinavir to the extent of 0.4%.

### Example 6. Assay performance with serum milieu

This example comprises repeating Example 5 with some modifications. The drug evaluated was saquinavir, and the drug diluent was 100% normal human serum. Therefore, the final assay was in 50% human serum, which reflects the addition of a half volume of the antiserum diluted in PBS-Tween. Additionally, 15 dilution steps were carried out as in the previous example with 7 dilution steps and indicated that the lowest non-zero drug concentration in Example 5 provided about a 50% inhibition with respect to zero drug. More dilution steps were therefore required to clarify the entire inhibition curve. All drug concentrations were carried out in triplicate. The data was charted using the average of the three wells at each drug concentration and error bars to obtain the dose response curve shown in Figure 2. As can be seen from the graph, the range of the assay is from less than a nanogram to 1 microgram per milliliter. The lowest detectable level is estimated to be 0.5 ng/ ml.

This example demonstrates the usage of an antiserum raised to saquinavir-KLH for determining the amount of drug in human serum via an enzyme-linked immunosorbent assay.

## Claims

1. An immunoassay for determining an HIV protease inhibitor in a sample comprising the steps of:
(a) combining a sample suspected of containing said protease inhibitor with a receptor specific for said inhibitor and a conjugate comprising a ligand of said inhibitor and a non-isotopic signal generating moiety,
(b) measuring the amount of said receptor bound to said conjugate by monitoring the production of signal generated by said moiety, and
(c) correlating said production of signal with the presence or amount of said inhibitor in said sample,
wherein said protease inhibitor is selected from the group consisting of saquinavir, amprenavir, indinavir, nelfinavir and ritonavir.

2. The method of claim 1, wherein said receptor is selected from the group consisting of antibodies and antibody fragments.

3. The method of claim 1-2, wherein said receptor is bound either directly or indirectly to a solid phase.

4. The method of claims 1-3, wherein said signal generating moiety is selected from the group consisting of enzymes, fluorogenic compounds, chemiluminescent materials, electrochemical mediators, particles, reporter groups, enzyme inhibitors, and polypeptide carriers.

5. A non-isotopic immunoassay for determining an HIV protease inhibitor in a sample comprising the steps of:
(a) combining a sample suspected of containing said protease inhibitor with a conjugate comprising a ligand of said protease inhibitor and mycophenolic acid, a receptor specific for said protease inhibitor, inosine-5' monophospate (IMP), nicotinamide adenine dinucleotide (NAD) and inosine-5'-monophosphate dehydrogenase (IMPDH)
(b) monitoring the production of reduced nicotinamide adenine dinucleotide, (NADH) and
(c) correlating the production of reduced nicotinamide adenine dinucleotide (NADH) with the presence or amount of said protease inhibitor in said sample,
wherein said protease inhibitor is selected from the group consisting of saquinavir, amprenavir, indinavir, nelfinavir and ritonavir.

6. A test kit for determining an HIV protease inhibitor in a sample comprising in packaged combination:
(a) a receptor specific for said inhibitor and
(b) a conjugate comprising a ligand of said inhibitor and a non-isotopic signal generating moiety,
wherein said protease inhibitor is selected from the group consisting of saquinavir, amprenavir, indinavir, nelfmavir and ritonavir and
wherein said signal generating moiety is selected from the group consisting of enzymes, fluorogenic compounds, chemiluminescent materials, electrochemical mediators, particles, reporter groups, enzyme inhibitors, and polypeptide carriers.

7. The test kit of claim 6, wherein said receptor is selected from the group consisting of antibodies and antibody fragments.

8. The test kit of claim 6-7, wherein said receptor is bound either directly or indirectly to a solid phase.

## Patentansprüche

1. Immunoassay zur Bestimmung eines HIV-Protease-Hemmstoffs in einer Probe, bei dem man die folgenden Schritte ausführt:
(a) Kombinieren einer vermutlich den Protease-Hemmstoff enthaltenden Probe mit einem für den Hemmstoff spezifischen Rezeptor und einem einen Liganden des Hemmstoffs sowie eine nichtisotope signalerzeugende Gruppierung umfassenden Konjugat;
(b) Messen der Menge des an das Konjugat gebundenen Rezeptors durch Verfolgen der von der Gruppierung erzeugten Signalproduktion; und
(c) Korrelieren der Signalproduktion mit dem Vorliegen bzw. der Menge des Hemmstoffs in der Probe,
wobei der Protease-Hemmstoff aus der Gruppe Saquinavir, Amprenavir, Indinavir, Nelfinavir und Ritonavir ausgewählt ist.

2. Verfahren nach Anspruch 1, wobei der Rezeptor aus der Gruppe Antikörper und Antikörperfragmente ausgewählt ist.

3. Verfahren nach Anspruch 1-2, wobei der Rezeptor entweder direkt oder indirekt an eine Festphase gebunden ist.

4. Verfahren nach Anspruch 1-3, wobei die signalerzeugende Gruppierung aus der Gruppe Enzyme, fluorogene Verbindungen, Chemilumineszenzmaterialien, elektrochemische Vermittler, Partikel, Reportergruppen, Enzymhemmstoffe und Polypeptidträgerstoffe ausgewählt ist.

5. Nichtisotoper Immunoassay zur Bestimmung eines HIV-Protease-Hemmstoffs in einer Probe, bei dem man die folgenden Schritte ausführt:
(a) Kombinieren einer vermutlich den Protease-Hemmstoff enthaltenden Probe mit einem einen Liganden des Protease-Hemmstoffs sowie Mycophenolsäure umfassenden Konjugat, einem für den Protease-Hemmstoff spezifischen Rezeptor, Inosin-5'-monophosphat (IMP), Nikotinamid-Adenin-Dinukleotid (NAD) und Inosin-5'-monophosphat-Dehydrogenase (IMPDH);
(b) Verfolgen der Produktion von reduziertem Nikotinamid-Adenin-Dinukleotid (NADH); und
(c) Korrelieren der Produktion von reduziertem Nikotinamid-Adenin-Dinukleotid (NADH) mit dem Vorliegen bzw. der Menge des Protease-Hemmstoffs in der Probe,
wobei der Protease-Hemmstoff aus der Gruppe Saquinavir, Amprenavir, Indinavir, Nelfinavir und Ritonavir ausgewählt ist.

6. Testkit zur Bestimmung eines HIV-Protease-Hemmstoffs in einer Probe, der in abgepackter Kombination Folgendes umfasst:
(a) einen für den Hemmstoff spezifischen Rezeptor und
(b) ein einen Liganden des Hemmstoffs sowie eine nichtisotope signalerzeugende Gruppierung umfassendes Konjugat,
wobei der Protease-Hemmstoff aus der Gruppe Saquinavir, Amprenavir, Indinavir, Nelfinavir und Ritonavir ausgewählt ist und
wobei die signalerzeugende Gruppierung aus der Gruppe Enzyme, fluorogene Verbindungen, Chemilumineszenzmaterialien, elektrochemische Vermittler, Partikel, Reportergruppen, Enzymhemmstoffe und Polypeptidträgerstoffe ausgewählt ist.

7. Testkit nach Anspruch 6, wobei der Rezeptor aus der Gruppe Antikörper und Antikörperfragmente ausgewählt ist.

8. Testkit nach Anspruch 6-7, wobei der Rezeptor entweder direkt oder indirekt an eine Festphase gebunden ist.

## Revendications

1. Immuno-essai pour déterminer un inhibiteur de la protéase de VIH dans un échantillon comprenant les étapes consistant à :
(a) combiner un échantillon soupçonné de contenir ledit inhibiteur de protéase avec un récepteur spécifique pour ledit inhibiteur et un conjugué comprenant un ligand dudit inhibiteur et une fraction non-isotopique produisant un signal,
(b) mesurer la quantité dudit récepteur lié audit conjugué en contrôlant la production de signal produit par ladite fraction, et
(c) corréler ladite production de signal avec la présence ou la quantité dudit inhibiteur dans ledit échantillon,
dans lequel ledit inhibiteur de protéase est choisi dans le groupe formé par le saquinavir, l'amprénavir, l'indinavir, le nelfinavir, et le ritonavir.

2. Procédé selon la revendication 1, dans lequel ledit récepteur est choisi dans le groupe formé par les anticorps et les fragments d'anticorps.

3. Procédé selon la revendication 1 à 2, dans lequel ledit récepteur est lié directement ou indirectement à une phase solide.

4. Procédé selon les revendications 1 à 3, dans lequel ladite fraction produisant un signal est choisie dans le groupe formé par les enzymes, les composés fluorogènes, les matières chimioluminescences, les médiateurs électrochimiques, les particules, les groupes rapporteurs, les inhibiteurs d'enzymes, et les véhicules polypeptidiques.

5. Immuno-essai non isotopique pour déterminer un inhibiteur de protéase de VIH dans un échantillon, comprenant les étapes consistant :
(a) à combiner un échantillon soupçonné de contenir ledit inhibiteur de protéase avec un conjugué comprenant un ligand dudit inhibiteur de protéase et de l'acide mycophénolique, un récepteur spécifique pour ledit inhibiteur de protéase, de l'inosine-5'-monophosphate (IMP), du nicotinamide adénine dinucléotide (NAD) et de l'inosine-5'-monophosphate déshydrogénase (IMPDH)
(b) à contrôler la production de nicotinamide adénine dinucléotide (NADH) réduite, et
(c) à corréler la production de nicotinamide adénine dinucléotide (NADH) réduite avec la présence ou la quantité dudit inhibiteur de protéase dans ledit échantillon,
dans lequel ledit inhibiteur de protéase est choisi dans le groupe formé par le saquinavir, l'amprénavir, l'indinavir, le nelfinavir et le ritonavir.

6. Nécessaire d'essai pour déterminer un inhibiteur de protéase de VIH dans un échantillon comprenant dans une combinaison emballée :
(a) un récepteur spécifique audit inhibiteur et
(b) un conjugué comprenant un ligand dudit inhibiteur et une fraction non-isotopique produisant un signal,
dans lequel ledit inhibiteur de protéase est choisi dans le groupe formé par le saquinavir, l'amprénavir, l'indinavir, le nelfinavir et le ritonavir et
dans lequel ladite fraction produisant un signal est choisie dans le groupe formé par les enzymes, les composés fluorogènes, les matières chimioluminescentes, les médiateurs d'électrochimie, les particules, les groupes rapporteurs, les inhibiteurs d'enzyme et les véhicules polypeptidiques.

7. Nécessaire d'essai selon la revendication 6, dans lequel ledit récepteur est choisi dans le groupe formé par les anticorps et les fragments d'anticorps.

8. Nécessaire d'essai selon la revendication 6 ou 7, dans lequel ledit récepteur est lié directement ou indirectement à une phase solide.
